(19) Europäisches Patentamt
European Patent Office
Office européen des brevets

(11) Veröffentlichungsnummer: **0 086 390**
**B1**

(12) # EUROPÄISCHE PATENTSCHRIFT

(45) Veröffentlichungstag der Patentschrift: **27.12.85**

(51) Int. Cl.⁴: **C 07 C 125/065**

(21) Anmeldenummer: **83100890.9**

(22) Anmeldetag: **01.02.83**

(54) **Verfahren zur Herstellung von N-substituierten Carbamaten.**

(30) Priorität: **11.02.82 DE 3204711**

(43) Veröffentlichungstag der Anmeldung:
**24.08.83 Patentblatt 83/34**

(45) Bekanntmachung des Hinweises auf die Patenterteilung:
**27.12.85 Patentblatt 85/52**

(84) Benannte Vertragsstaaten:
**BE CH DE FR GB IT LI NL**

(56) Entgegenhaltungen:
**DE - B - 1 157 598**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Nestler, Gerhard, Dr., Van-Leyden-Strasse 17, D-6700 Ludwigshafen (DE)**

**EP 0 086 390 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zur Herstellung von N-substituierten Carbamaten durch Umsetzung von Carbaminsäureestern mit Olefinen in Gegenwart von sulfonsauren Kationenaustauschern und in Gegenwart von 0,1 bis 50 Gramm Alkohol je Mol Ausgangsstoff II.

Aus der DE-AS 1 157 598 ist bekannt, daß die Umsetzung von Carbaminsäureestern mit Olefinen in Gegenwart von wasserfreien Mineralsäuren, organischen Sulfonsäuren oder Lewissäuren N-alkylierte Carbaminsäureester liefert. Bevorzugt werden dabei konzentrierte Schwefelsäure und Bortrifluoridetherat eingesetzt (loc. cit., Spalte 2, Seiten 22—25 und Beispiele 1—12). Nachteilig für dieses Verfahren ist jedoch die notwendige aufwendige und abwasserbelastende Abtrennung des Katalysators, das Auftreten von Nebenprodukten (Telomere der Olefine) und die dadurch bedingte geringe Ausbeute (10 bis 70% der Theorie). Zwar wird die Gruppe saurer Ionenaustauscher erwähnt, aber weder Einzelstoffe noch Arbeitsweise und Ausführungsbeispiele beschrieben. Als Lösungsmittel werden Chlorkohlenwasserstoffe, aromatische oder aliphatische Kohlenwasserstoffe erwähnt, in allen Beispielen aber nur Benzol und Toluol und Eisessig verwendet. Es ist ebenso bekannt, daß es unter den stark sauren Alkylierungsbedingungen zu einer teilweisen Telomerisation der eingesetzten Olefine kommt. Diese Telomere können sich wieder mit dem eingesetzten Carbaminsäureester zu N-alkylierten Carbamaten umsetzen (DE-AS 1 157 598, Spalte 3, Zeilen 7—10).

In der DE-OS 2 925 480 wird ein Verfahren zur Herstellung von p-substituierten, aromatischen Carbaminsäureestern durch Umsetzung von aromatischen Carbaminsäureestern mit Olefinen in Gegenwart anorganischer Säuren oder Sulfonsäuren beschrieben. Auch sulfonsaure Kationenaustauscher werden näher beschrieben und in Beispielen verwendet. Die Carbaminsäureester haben stets einen aromatischen Rest als Substituenten am Stickstoffatom, das Stickstoffatom kann disubstituiert oder monosubstituiert sein. Alle Beispiele zeigen einen monosubstituierten N-Phenylcarbaminsäureester. Das verwendete Olefin greift nicht am Stickstoffatom an, sondern substituiert den aromatischen Rest, z. B. Phenylrest, in p-Stellung. Es wird beschrieben, daß in der Regel keine Lösungsmittel verwendet werden; Alkohole werden nicht erwähnt.

Es wurde nun gefunden, daß man N-substituierte Carbamate der Formel

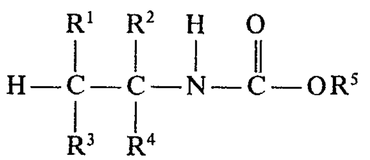

(I)

worin die einzelnen Reste $R^1$, $R^2$ und $R^3$ gleich oder verschieden sein können und jeweils mindestens der Rest $R^2$ von diesen Resten einen aliphatischen, cycloaliphatischen, araliphatischen oder aromatischen Rest bedeutet, $R^4$ einen aliphatischen, cycloaliphatischen oder araliphatischen Rest bezeichnet oder, wenn $R^2$ für einen aromatischen Rest oder für ein Glied eines alicyclischen, bicyclischen oder tricyclischen Restes steht, auch ein Wasserstoffatom bezeichnet, jeweils mindestens 2 der Reste $R^1$, $R^2$, $R^3$ und $R^4$ auch für Glieder eines alicyclischen, bicyclischen oder tricyclischen Restes stehen, $R^1$ und/oder $R^3$ auch ein Wasserstoffatom bedeuten, $R^5$ einen aliphatischen, cycloaliphatischen oder araliphatischen Rest bezeichnet, durch Umsetzung von Carbaminsäureestern mit Olefinen in Gegenwart von sulfonsauren Kationenaustauschern als Katalysatoren vorteilhaft erhält, wenn man Carbaminsäureester der Formel

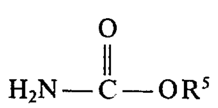

(II)

worin $R^5$ die vorgenannte Bedeutung besitzt, mit Olefinen der Formel

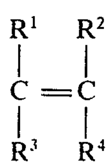

(III)

worin $R^1$, $R^2$, $R^3$ und $R^4$ die vorgenannte Bedeutung besitzen, in Gegenwart von 0,1 bis 50 Gramm Alkohol je Mol Ausgangsstoff II umsetzt.

Die Umsetzung kann für den Fall der Verwendung des Carbaminsäureethylesters und Isobuten durch die folgenden Formeln wiedergegeben werden:

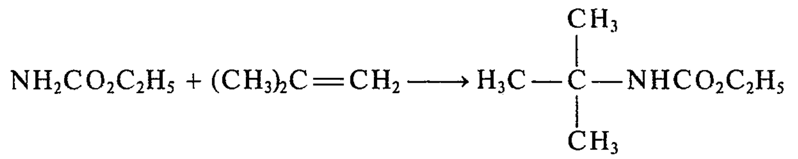

Im Vergleich zu den bekannten Verfahren liefert das Verfahren nach der Erfindung überraschend auf einfacherem und wirtschaftlicherem Wege N-substituierte Carbaminsäureester in besserer Ausbeute, Raum-Zeit-Ausbeute und Reinheit, gerade auch im großtechnischen Maßstab. Im Gegensatz zu den Umsetzungen entsprechend der DE-AS 1 157 598 (siehe Vergleichsbeispiele 1 und 2) wird bei der erfindungsgemäßen Alkylierung in Gegenwart eines Alkohols die Bildung von Olefinoligomeren und der entsprechenden N-substituierten Carbaminsäureester weitgehend verhindert. Es ist außerdem überraschend, daß es dabei weder zur Bildung von Carbonaten noch zur Desaktivierung (Neutralisation) des Katalysators durch freiwerdende Amine kommt.

Entsprechend dem Stand der Technik (»The Chemistry of Open-Chain Nitrogen Compounds« Vol. 1, Seite 261 (Gleichung 107), W. A. Benjamin Inc., 1965, New York), hätte man nämlich anstelle der dort beschriebenen sauren Hydrolyse eine säurekatalysierte Alkoholyse der Carbamate entsprechend der folgenden Gleichungen erwartet:

$$NH_2CO_2R^7 + R^6OH \longrightarrow NH_3 + R^6OCO_2R^7$$

$$R^8NHCO_2R^7 + R^6OH \longrightarrow R^8NH_2 + R^6OCO_2R^7$$

Wie die Beispiele 5—9 zeigen, konnte jedoch weder eine Desaktivierung des Katalysators festgestellt werden, noch die Bildung von Carbonaten nachgewiesen werden.

Es wurde außerdem überraschend gefunden, daß im Gegensatz zur DE-AS 1 157 598 (Spalte 1, Zeile 38) n-Alkene unter den erfindungsgemäßen Bedingungen praktisch nicht reagieren und man daher beispielsweise bei der Herstellung von N-tert.-Butylcarbamaten den wesentlich kostengünstigeren $C_4$-Schnitt verwenden kann. Ein weiterer Vorteil des Verfahrens besteht darin, daß der Katalysator äußerst einfach und ohne Belastung der Umwelt abgetrennt werden kann und beliebig oft wiederverwendet werden kann.

In der DE-AS 1 157 598 (Spalte 2, Zeile 19) wird zwar die Verwendung von sauren Ionenaustauschern vorgeschlagen, doch da keine Angaben über den Austauscher gemacht werden, mußte angenommen werden, daß es sich dabei um die zu dieser Zeit üblichen Austauscherharze vom Gel-Typ handelt. Wie jedoch Vergleichsbeispiel 1 zeigt, lassen sich mit diesen Austauscherharzen vom Gel-Typ nur unbefriedigende Ergebnisse erzielen. Es ist überraschend, daß solche Gelkatalysatoren bei dem erfindungsgemäßen Verfahren gute Ergebnisse liefern.

Das Olefin III kann mit dem Carbaminsäureester II in stöchiometrischer Menge, im Überschuß oder Unterschuß, vorzugsweise in einem Verhältnis von 0,3 bis 5, insbesondere 0,5 bis 4 Mol Olefin III je Mol Carbaminsäureester II umgesetzt werden. Bevorzugte Carbaminsäureester II, Olefine III und dementsprechend bevorzugte N-substituierte Carbamate I sind solche, in deren Formeln die einzelnen Reste $R^1$, $R^2$ und $R^3$ gleich oder verschieden sein können und jeweils mindestens der Rest $R^2$ von diesen Resten einen Alkylrest mit1 bis 10, insbesondere 1 bis 4 Kohlenstoffatomen, einen gegebenenfalls mit Alkylgruppen, insbesondere 1 oder 2 Alkylgruppen mit jeweils 1 bis 4 Kohlenstoffatomen substituierten Cycloalkylrest mit 5 bis 8 Kohlenstoffatomen, einen Aralkylrest mit 7 bis 12 Kohlenstoffatomen oder einen Phenylrest bedeutet, $R^4$ einen Alkylrest mit 1 bis 10, insbesondere 1 bis 4 Kohlenstoffatomen, einen gegebenenfalls mit Alkylgruppen, insbesondere 1 oder 2 Alkylgruppen mit jeweils 1 bis 4 Kohlenstoffatomen substituierten Cycloalkylrest mit 5 bis 8 Kohlenstoffatomen, einen Aralkylrest oder Alkylarylrest mit 7 bis 12 Kohlenstoffatomen bezeichnet oder, wenn $R^2$ für einen aromatischen Rest oder für ein Glied eines alicyclischen, bicyclischen oder tricyclischen Restes steht, auch ein Wasserstoffatom bezeichnet, jeweils mindestens 2 der Reste $R^1$, $R^2$, $R^3$ und $R^4$ auch für Glieder eines alicyclischen Ringes mit 5 bis 8 Kohlenstoffatomen oder eines bicyclischen Ringes mit 7 bis 10 Kohlenstoffatomen oder eines tricyclischen Ringes mit 10 bis 12 Kohlenstoffatomen stehen, $R^1$ und/oder $R^3$ auch ein Wasserstoffatom bedeuten, $R^5$ einen Alkylrest mit 1 bis 10, insbesondere 1 bis 8 Kohlenstoffatomen, einen Cycloalkylrest mit 5 bis 8 Kohlenstoffatomen, einen Aralkylrest mit 7 bis 12 Kohlenstoffatomen bezeichnet. Die aliphatischen Olefine III sind stets verzweigt, wobei sowohl $R^2$ als auch $R^4$ einen Alkylrest bezeichnen. Die vorgenannten Reste und Ringe können noch durch unter den Reaktionsbedingungen inerte Gruppen oder Atome, z. B. Alkylgruppen oder Alkoxygruppen mit 1 bis 5 Kohlenstoffatomen, Chlor- oder Bromatome, substituiert sein. Die Olefine II können gegebenenfalls 2 Doppelbindungen tragen, z. B. Isopren, Limonen, Dicyclopentadien; in der Regel setzt sich nur eine Doppelbindung des Olefins erfindungsgemäß um.

Bevorzugt verwendet man verzweigte Alkene. Es können im Gegensatz zu den bekannten Verfahren vorteilhaft auch Gemische von Alkenen und gegebenenfalls von Alkenen mit Alkanen verwendet werden, wie sie z. B. bei der Crackung oder Dehydrierung von Kohlenwasserstoffen, z. B. Erdöl, oder der Oligomerisierung von Olefinen, insbesondere von Isobutylen, Propylen oder n-Buten, oder der Kohlenoxidhydrierung entstehen.

Es können z. B. folgende Olefine als Ausgangsstoffe II verwendet werden: 2-Methylpenten-1, 2-Ethylpenten-1, 2-Propylpenten-1, 2-Methylhexen-1, 2-Ethylhexen-1; vorgenannte Alkene, die in 1-Stellung oder 3-Stellung oder 4-Stellung durch die Methyl-, Ethyl-, n-Propyl-, Isopropyl-, n-Butyl-, Isobutyl-, sek.-Butyl-, tert.-Butyl-Gruppe substituiert sind; 2,3,3-Trimethylbuten-1, 2-Ethyl-3,3-dimethyl-buten-1, 2,3,3-Trimethylhepten-(1), 2,4,4-Trimethylpenten-(1), 2,3,3-Trimethylpenten-(1), 2,3,4-Trimethylpenten-(1); analoge Alkene, deren Doppelbindung in 2-Stellung im Molekül liegen; verzweigte Alkene, wie sie in Gestalt von Gemischen bei der Dimerisierung von Isobutylen oder n-Buten (Octene) oder Trimerisierung von Isobutylen oder n-Buten (Dodecene) oder Propylen (Nonene) bzw. Tetramerisierung von Propylen (Dodecene) anfallen; $\alpha$-Methylstyrol, o-Methylstyrol, m-Methylstyrol, p-Methylstyrol, 3,5-Dimethylstyrol, o-Ethylstyrol, m-Ethylstyrol, p-Ethylstyrol, 4-Chlor-styrol; Cyclohepten, 1-Methylcyclohexen-1, Benzonorbornen.

Bevorzugt sind: Isobutylen, Styrol, 2,3-Dimethylbuten-1, 2-Methylbuten-1, 2-Methylbuten-2, 2-Ethylbuten-1, Cyclopenten, Cyclohexen, $\alpha$-Methylstyrol, Norbornen, 2-Ethylhexen-1, 2-Ethylhexen-2, Diisobuten(2,4,4-Trimethylpenten-1), 1-Methylcyclohexen-1, Camphen, Dicyclopentadien.

Beispielsweise sind folgende Carbaminsäureester II geeignet: Carbaminsäuremethylester; homologe Ethyl-, Propyl-, Isopropyl-, Benzyl-, Butyl-, Isobutyl-, Octyl-, Cyclohexyl-, Cyclopentyl-, n-Hexyl-ester.

Der Alkohol wird in einer Menge von 0,1 bis 50, insbesondere 0,5 bis 20 Gramm pro Mol Carbaminsäureester II verwendet. Als Alkohole kommen solche der Formel

$$R^5OH \qquad (IV)$$

worin $R^5$ die vorgenannte allgemeine und bevorzugte Bedeutung besitzt, in Betracht. Es wird vorzugsweise der dem Carbaminsäureester II zugrundeliegende Alkohol verwendet.

Die Umsetzung wird im allgemeinen bei einer Temperatur von 25 bis 160°C, vorzugsweise von 40 bis 150°C, insbesondere 60 bis 130°C, mit Unterdruck, Überdruck oder zweckmäßig drucklos, kontinuierlich oder diskontinuierlich durchgeführt. Die Verweilzeit beträgt vorzugsweise von 0,5 bis 10, insbesondere von 0,5 bis 5 Stunden. Zweckmäßig verwendet man keine zusätzlichen Lösungsmittel.

Als Katalysatoren werden organische, sulfonsaure Kationenaustauscher, vorteilhaft Harze aus sulfoniertem Polystyroldivinylbenzol oder sulfonierten, vernetzten Styrolpolymeren; sulfonsaure Phenolformaldehyd- oder Benzolformaldehydharze oder Copolymerisate aus Tetrafluorethylen und Vinylsulfonylfluorid verwendet. Vorzugsweise kommen sulfonierte Polystyroldivinylbenzolaustauscher in Frage. Die Austauscher liegen in der Säureform und nicht als Salz vor. Der Katalysator kann sowohl makroporöse als auch gelartige Struktur besitzen. Er hat vorteilhaft eine Korngröße von 5 bis 2000, vorzugsweise von 10 bis 1500, im Falle von gelartigen Katalysatoren insbesondere von 10 bis 200 Mikrometer und eine Gesamtoberfläche von 10 bis 300 $m^2/g$, im Falle der makroporösen Katalysatoren insbesondere eine innere Oberfläche von 10 bis 200 $m^2/g$. Geeignet sind z. B. Austauscherharze, wie sie unter der Bezeichnung ®LEWASORB AC-10, ®Amberlyst-15, ®LEWATIT SPC-118, ®LEWATIT SPC-108 oder ®Nafion im Handel erhältlich sind. Man entwässert sie zweckmäßig vor der Verwendung in üblicher Weise, z. B. durch Erhitzen auf 100°C bis 110°C im Vakuum. Die Entwässerung kann jedoch auch durch Verdrängen mit hydrophilen organischen Flüssigkeiten und anschließendem Erhitzen auf 100°C bei vermindertem Druck oder durch azeotrope Destillation mit einer organischen Flüssigkeit erfolgen.

Der Katalysator in Gestalt eines Ionenaustauschers kann in beliebiger diskontinuierlicher oder kontinuierlicher Arbeitsweise, z. B. in Gestalt eines Festbettes, verwendet werden. Er befindet sich vorteilhaft während der Umsetzung in Suspension, in der Regel im sich bildenden Reaktionsgemisch. Zweckmäßig legt man einen Anteil an flüssigem Carbaminsäureester II und Olefin III vor und suspendiert den Katalysator in dem Gemisch unter guter Durchmischung. Bevorzugt ist eine Menge von 3 bis 100, insbesondere von 5 bis 50 Gramm Ionenaustauscher, bezogen auf 1 Mol Carbaminsäureester II. Zweckmäßig wird das Reaktionsgemisch während der gesamten Umsetzung durchmischt, vorzugsweise mit einer Rührgeschwindigkeit von mindestens 100, zweckmäßig von 200 bis 2000, insbesondere von 300 bis 1000 Umdrehungen pro Minute. Bei Durchmischungseinrichtungen ohne Rührwerk, z. B. auch bei Durchmischung mittels Inertgas wie Stickstoff, werden solche bevorzugt, die vorgenannter Rührgeschwindigkeit entsprechende Scherenergie einbringen. Auf diese Weise wird eine feindisperse Suspension erzielt.

Die Reaktion kann wie folgt durchgeführt werden: Ein Gemisch von Ausgangsstoff II und III, Katalysator und Alkohol IV wird während der Reaktionszeit bei der Reaktionstemperatur gehalten. Nach Abtrennung des Katalysators kann man den Endstoff in üblicher Weise, z. B. durch Filtration und Destillation, isolieren.

Die Reaktion kann im Falle der Verwendung von Ionenaustauschern als Katalysatoren und im kontinuierlichen Betrieb im Festbett oder vorteilhaft wie folgt durchgeführt werden: Ein flüssiges Gemisch von Carbaminsäureester, Olefin und Alkohol, wird bei der Reaktionstemperatur und dem Reaktionsdruck durch eine Suspension des Katalysators im Ausgangsgemisch bzw. Reaktionsgemisch geleitet und filtriert. Dann wird aus dem Reaktionsgemisch der Endstoff in üblicher Weise, z. B. durch Destillation, abgetrennt. Die Filtrierung erfolgt zweckmäßig vor Austritt der Suspension aus dem Reaktor. Als Filter kommen säurefeste Filtertücher, Drahtnetzfilter, Sintermetallfilter, sofern die Maschenweiten bzw. Porendurchmesser kleiner als die Katalysatorpartikel sind, in Betracht.

Die nach dem Verfahren der Erfindung herstellbaren N-substituierten Carbamate I sind Wirkstoffe und wertvolle Ausgangsstoffe für die Herstellung von Farbstoffen, Schädlingsbekämpfungsmitteln und Pharmazeutika. Durch Hydrolyse der Carbaminsäureester (Houben-Weyl, Band 11/1, Seiten 448—952) können die entsprechenden Amine hergestellt werden, die ebenfalls wichtige Ausgangsprodukte bei der Synthese von Wirkstoffen darstellen. Bezüglich der Verwendung wird auf die vorgenannten Veröffentlichungen und Ullmanns Encyklopädie der technischen Chemie, Band 5, Seiten 73 bis 76, verwiesen.

Beispiel 1

In einem Rührautoklaven wurde ein Gemisch aus 89 Gramm Carbaminsäureethylester, 5 Gramm Ethanol, 10 Gramm ®LEWASORB AC-10 und 70 Gramm Isobuten unter Rühren auf 70°C erhitzt und 4 Stunden bei dieser Temperatur gerührt. Nach Beendigung der Umsetzung wurde der Katalysator abgetrennt und destillativ aufgearbeitet. Man erhielt 136 Gramm (93% der Theorie) N-tert.-Butylcarbaminsäureethylester vom Kp 70—71°C/20 mbar. Der gaschromatographisch ermittelte Gehalt an Diisobuten im Rohprodukt betrug 0,2 Gewichtsprozent, Diethylcarbonat konnte dagegen nicht nachgewiesen werden.

Beispiel 2

In einem Rührautoklaven wurde ein Gemisch aus 117 Gramm Carbaminsäurebutylester, 5 Gramm Butanol, 15 Gramm ®LEWASORB AC-10 und 60 Gramm Isobuten unter Rühren auf 70°C erhitzt und 4 Stunden bei dieser Temperatur gerührt. Anschließend wurde der Katalysator abgetrennt und das Gemisch im Vakuum destilliert. Man erhielt 161 Gramm (93% der Theorie) N-tert.-Butylcarbaminsäurebutylester vom Kp 110—111°C/20 mbar. Diisobuten wurde nicht in deutlicher Menge beobachtet.

Beispiele 3—7

Der in Beispiel 2 eingesetzte Katalysator wurde in den folgenden, entsprechend Beispiel 2 durchgeführten Umsetzungen wiederverwendet.

| | Ausbeute (% der Theorie) |
|---|---|
| Beispiel 3 | 94 |
| Beispiel 4 | 89 |
| Beispiel 5 | 90 |
| Beispiel 6 | 93 |
| Beispiel 7 | 92 |

Der Gehalt an Diisobuten betrug 0—0,2 Gewichtsprozent; Carbonate konnten nicht nachgewiesen werden.

Beispiel 8

In einem Rührautoklaven wurde ein Gemisch aus 117 Gramm Carbaminsäurebutylester, 5 Gramm Butanol, 17 Gramm LEWATIT SPC-118 und 160 Gramm eines Gemisches aus 49 Gewichtsprozent Isobuten und 41,5 Gewichtsprozent n-Butene und 9,5 Gewichtsprozent Butane unter Rühren auf 70°C erhitzt und 5 Stunden bei dieser Temperatur gerührt. Anschließend wurde der Katalysator abgetrennt und das Gemisch im Vakuum destilliert. Man erhielt 149 Gramm (86% der Theorie) N-tert.-Butylcarbaminsäurebutylester vom Kp 110—111°C/20 mbar. Diisobuten wurde nicht in deutlicher Menge beobachtet.

LEWATIT SPC-118 ist ein im Handel erhältlicher, makroporöser, sulfonsaurer Kationenaustauscher aus Styrol-Divinylbenzol (18%)-Co-polymerisat mit einer Korngröße von 30 bis 1500 Mikrometer, einer inneren Oberfläche von 40 $m^2/g$.

Beispiel 9

In einem Rührreaktor wurde ein Gemisch aus 178 Gramm Carbaminsäureethylester, 94 Gramm Norbornen, 5 Gramm Ethanol und 20 Gramm LEWASORB AC-10 unter Rühren auf 70°C erhitzt und

7 Stunden bei dieser Temperatur gerührt. Anschließend wurde der Katalysator abfiltriert und das Gemisch im Vakuum destilliert. Man erhielt 157 Gramm (86% der Theorie) N-Norbornylcarbaminsäureethylester vom Kp 148—150°C/20 mbar bzw. vom Fp 50°C (aus Pentan). Oligomere des Norbornens werden nicht in deutlicher Menge beobachtet.

Beispiel 10

(Vergleichsbeispiel)

In einem Rührautoklaven wurde ein Gemisch aus 89 Gramm Carbaminsäureethylester, 10 Gramm ®LEWATIT SC-108 und 70 Gramm Isobuten unter Rühren auf 70°C erhitzt und 4 Stunden bei dieser Temperatur gerührt. Nach Beendigung der Umsetzung wurde der Katalysator abgetrennt und das Reaktionsgemisch gaschromatographisch analysiert Es enthielt neben den unumgesetzten Ausgangsstoffen 41 Gramm (28% der Theorie) N-tert.-Butylcarbaminsäureethylester und 2 Gramm Diisobuten.

LEWATIT SC-108 ist ein im Handel erhältlicher, gelförmiger, sulfonsaurer Kationenaustauscher aus Styrol-Divinylbenzol (8%)-Co-polymerisat mit einer Korngröße von 300 bis 1500 Mikrometer.

Beispiel 11

(Vergleichsbeispiel)

In einem Rührautoklaven wurde ein Gemisch aus 89 Gramm Carbaminsäureethylester, 10 Gramm ®LEWASORB AC-10 und 70 Gramm Isobuten unter Rühren auf 70°C erhitzt und 4 Stunden bei dieser Temperatur gerührt. Nach Beendigung der Umsetzung wurde der Katalysator abgetrennt und das Reaktionsgemisch gaschromatographisch analysiert. Es enthielt neben dem Endstoff noch 4,5 Gewichtsprozent Diisobuten. Durch Destillation des Rohproduktes erhielt man 134 Gramm (92% der Theorie) N-tert.-Butylcarbaminsäureethylester vom Kp 70—71°C/20 mbar.

LEWASORB AC-10 ist ein im Handel erhältlicher, gelförmiger, sulfonsaurer Kationenaustauscher aus Styrol-Divinylbenzol-(8%)-Co-polymerisat mit einer Korngröße von 10 bis 200 Mikrometer.

**Patentanspruch**

Verfahren zur Herstellung von N-substituierten Carbamaten der Formel

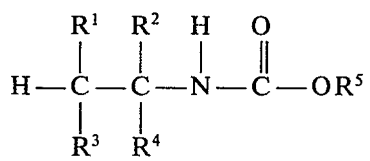

(I)

worin die einzelnen Reste $R^1$, $R^2$ und $R^3$ gleich oder verschieden sein können und jeweils mindestens der Rest $R^2$ von diesen Resten einen aliphatischen, cycloaliphatischen, araliphatischen oder aromatischen Rest bedeutet, $R^4$ einen aliphatischen, cycloaliphatischen oder araliphatischen Rest bezeichnet oder, wenn $R^2$ für einen aromatischen Rest oder für ein Glied eines alicyclischen, bicyclischen oder tricyclischen Restes steht, auch ein Wasserstoffatom bezeichnet, jeweils mindestens 2 der Reste $R^1$, $R^2$, $R^3$ und $R^4$ auch für Glieder eines alicyclischen, bicyclischen oder tricyclischen Restes stehen, $R^1$ und/oder $R^3$ auch ein Wasserstoffatom bedeuten, $R^5$ einen aliphatischen, cycloaliphatischen oder araliphatischen Rest bezeichnet, durch Umsetzung von Carbaminsäureestern mit Olefinen in Gegenwart von sulfonsauren Kationenaustauschern als Katalysatoren, dadurch gekennzeichnet, daß man Carbaminsäureester der Formel

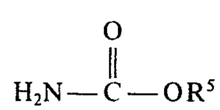

(II)

worin $R^5$ die vorgenannte Bedeutung besitzt, mit Olefinen der Formel

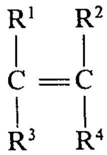

(III)

worin $R^1$, $R^2$, $R^3$ und $R^4$ die vorgenannte Bedeutung besitzen, in Gegenwart von 0,1 bis 50 Gramm Alkohol je Mol Ausgangsstoff II umsetzt.

**Claim**

A process for the preparation of an N-substituted carbamate of the formula

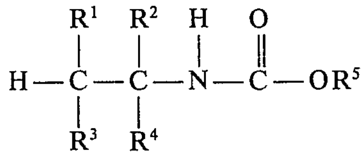

(I)

where the individual radicals $R^1$, $R^2$ and $R^3$ may be identical or different and $R^2$ is always an aliphatic, cycloaliphatic, araliphatic or aromatic radical, $R^4$ is an aliphatic, cycloaliphatic or araliphatic or, when $R^2$ is an aromatic radical or a member of an alicyclic, bicyclic or tricyclic radical, may furthermore be hydrogen, at least 2 of the radicals $R^1$, $R^2$, $R^3$ and $R^4$ may furthermore be members of an alicyclic, bicyclic or tricyclic radical, $R^1$ and/or $R^3$ may furthermore be hydrogen, and $R^5$ is an aliphatic, cycloaliphatic or araliphatic radical, by reaction of a carbamate with an olefin in the presence of a cation exchanger containing sulfonic acid groups, as the catalyst, wherein an carbamate of the formula

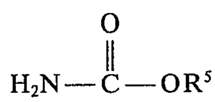

(II)

where $R^5$ has the above meanings, is reacted with an olefin of the formula

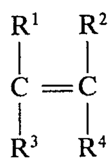

(III)

where $R^1$, $R^2$, $R^3$ and $R^4$ have the above meanings, in the presence of from 0.1 to 50 g of an alcohol per mole of starting material II.

**Revendication**

Procédé pour la préparation de carbamates N-substitués de formule

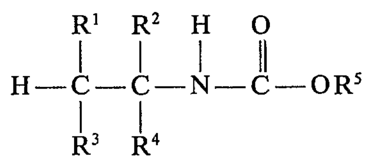

(I)

dans laquelle les différents radicaux $R^1$, $R^2$ et $R^3$ peuvent être semblables ou dissemblables et parmi lesquels le radical $R^2$ au moins désigne un radical aliphatique, cycloaliphatique, araliphatique ou aromatique, $R^4$ désigne un radical aliphatique, cycloaliphatique ou araliphatique ou, lorsque $R^2$ est mis pour un radical aromatique ou pour un terme d'un radical alicyclique, bicyclique ou tricyclique, désigne également un atome d'hydrogène, deux au moins des radicaux $R^1$, $R^2$, $R^3$ et $R^4$ sont également mis pour des termes d'un radical alicyclique, bicyclique ou tricyclique, $R^1$ et/ou $R^3$ représentent aussi un atome d'hydrogène, $R^5$ désigne un radical aliphatique, cycloaliphatique ou araliphatique, par réaction d'esters d'acide carbamique avec des oléfines en présence d'échangeurs de cations sulfoniques en tant que catalyseurs, caractérisé en ce qu'on fait réagir des esters d'acide carbamique de formule

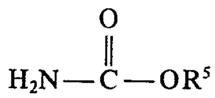

(II)

dans laquelle $R^5$ a la signification donnée ci-dessus, avec des oléfines de formule

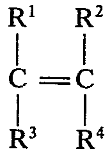

(III)

dans laquelle $R^1$, $R^2$, $R^3$ et $R^4$ ont les significations données ci-dessus, en présence de 0,1 à 50 g d'alcool par mol de substance de départ II.